# FASCICULE DE BREVET EUROPEEN

(11) **EP 2 674 754 B1**
(45) Date de publication et mention de la délivrance du brevet: **25.03.2015**
(21) Numéro de dépôt: 13171590.6
(22) Date de dépôt: 12.06.2013
(51) Int. Cl.: G01N 33/28, G01N 21/03

(54) **Cellule pour l'analyse sous très haute pression d'échantillons de fluides et procédé de mesure associé**
Zelle für die Analyse von Fluidproben unter sehr hohem Druck und entsprechendes Verfahren zur Analyze
Cell for analysing fluid samples under very high pressure and corresponding method of analysis

(30) Priorité: 12.06.2012 FR 1255465
(43) Date de publication de la demande: 18.12.2013
(73) Titulaire: Vinci Technologies, 92000 Nanterre (FR)
(72) Inventeur: Legrand, Stephane, 75008 Paris (FR); Manissol, Yannick, 77163 Dammartin sur Tigeaux (FR); Delahais, Jeremy, 78560 Le Pont Marly (FR); Decrossas, Philippe, 78390 Bois d'Arcy (FR)
(74) Mandataire: Bugnion Genève

(56) Documents cités:
- US-A1- 2008 016 944
- US-A1- 2008 252 881
- US-B2- 6 827 842
- CASTILLO J ET AL: "Optical fiber extrinsic refractometer to measure RI of samples in a high pressure and temperature systems: Application to wax and asphaltene precipitation measurements", FUEL, IPC SCIENCE AND TECHNOLOGY PRESS, GUILDFORD, GB, vol. 85, no. 14-15, 1 octobre 2006 (2006-10-01), pages 2220-2228, XP028064646, ISSN: 0016-2361, DOI: 10.1016/J.FUEL.2006.03.020 [extrait le 2006-10-01]

## Description

La présente invention concerne une cellule d'analyse sous très haute pression et température élevée d'échantillons de fluides et un procédé de détection et de mesure utilisant ladite cellule pour déterminer les conditions de formation de particules solides et, le cas échéant, mesurer la quantité de matières solides présente dans lesdits échantillons.

Une telle cellule trouve son application, notamment, dans l'analyse des propriétés thermodynamiques et physico-chimiques des hydrocarbures.

Ces cellules sont utilisées en laboratoire pour effectuer les tests et les mesures permettant de connaître la composition du pétrole brut prélevé sur un site de prospection avant son exploitation industrielle et commerciale.

Ces mesures sont plus particulièrement destinées à déterminer, de façon précise et parfois directement sur le site du gisement exploratoire, la présence éventuelle de matières solides et leur quantité dans le pétrole brut ainsi que le comportement thermodynamique de ces matières lors d'essais dits « PVT » c'est à dire, respectivement, en pression, volume et température.

La recherche d'un moyen de détermination et de prévision des conditions thermodynamiques susceptibles de conduire à la génération de particules solides dans des fluides pétroliers lors de leur exploitation, est devenue un enjeu majeur dans le domaine de l'exploration pétrolière.

On entend par formation de particules solides les phénomènes de floculation ou de précipitation d'asphaltènes, de formation de cristaux de paraffines (Wax) ou d'hydrates.

En effet, à haute pression dans les gisements, les asphaltènes sont dissous dans le pétrole brut qui est donc sous forme monophasique.

Cependant, lors de l'extraction du brut via le forage, la pression diminue en remontant vers la surface et le couple pression/température présente une première valeur critique appelée AOP (Asphaltènes Onset precipitation) qui correspond à la précipitation des asphaltènes. Il existe aussi une seconde valeur critique appelée WAT (Wax Appearance Temperature) à laquelle sont susceptibles d'apparaître des cristaux de paraffines. Pour chaque échantillon, il est possible de prévoir la mesure du point de bulle qui correspond, pour une température donnée, à la pression de changement d'état du fluide (liquide-gaz).

Ainsi, dès que l'équilibre est rompu, par des variations de température, de pression ou par une simple modification de la composition chimique, une phase solide se forme qui est susceptible de provoquer le bouchage ou l'encrassement accidentel des conduites du forage et/ou des pipelines ce qui est fortement préjudiciable pour l'exploitation du puits.

Il s'avère donc nécessaire de prévoir, à partir d'échantillons prélevés au fond du puits (dits « bottomhole samples »), le comportement ultérieur de ses composants asphaltènes/ paraffines/hydrates afin d'éviter leur impact négatif lors de toutes les étapes de la production, du transport et du raffinage des hydrocarbures et en vue d'adapter les installations et équipements qui y seront dédiés.

Ce besoin est, en outre, accru pour les dénommés « nouveaux pétroles » que sont, notamment, les schistes bitumineux et les huiles profondes extraites des puits « offshore ».

Ces hydrocarbures lourds et certaines huiles légères sont plus problématiques à exploiter et à transporter car le risque d'apparition de dépôts de particules solides, lors de la remontée dans les puits et dans les conduites de transport du fait des changements importants de pression et de température, est notablement plus élevé que pour les hydrocarbures traditionnels.

La prévision et/ou la neutralisation du risque de dépôts solides constitue donc un nouveau défi pour l'industrie pétrolière qui recherche, pour des raisons logistiques et donc économiques, des garanties de fluidité du pétrole lors de toutes les étapes de l'exploitation.

Par conséquent, l'analyse d'échantillons d'hydrocarbures, préalablement à l'exploitation industrielle du gisement ou dans le cadre de la conception de techniques de récupération (Enhanced Oil Recovery) s'avère désormais incontournable. Elle consiste à simuler et/ou à reproduire les conditions thermodynamiques auxquelles vont être soumis les hydrocarbures lors de leur extraction afin d'évaluer, en fonction de leur composition, les risques liés à l'apparition potentielle de phases solides.

Les méthodes d'analyse communément utilisées pour les fluides bi-phasiques sont fondées sur la mesure des propriétés optiques et, en particulier, de la diffraction et/ou de l'absorption par les particules solides au sein du fluide analysé.

Pour les fluides pétroliers, les mesures sont généralement effectuées soit au moyen d'un microscope, soit en éclairant l'échantillon au moyen d'un faisceau laser dans les longueurs d'onde de l'infrarouge et en mesurant la puissance optique transmise et/ou absorbée au travers dudit échantillon.

Le facteur d'absorption (lumière absorbée) ou réciproquement le facteur de transmission (lumière transmise) varie en fonction, à la fois, de la densité du fluide (liée aux conditions de pression et température) et de la présence (éventuellement aussi de la quantité) de particules solides (asphaltènes, par exemple).

En ce qui concerne l'équipement, les cellules d'analyse connues, comme celles décrites dans le US 2008/016944 et le US 2008/252881,comprennent généralement un cylindre dans lequel est logé un piston mobile en translation axiale qui délimite, avec les parois d'extrémité du cylindre, une chambre de compression dans laquelle l'échantillon est soumis à une très forte pression pendant qu'il est analysé optiquement.

Toutefois, dans le dispositif du US 2008/016944, les éléments de mesure sont fixes ce qui ne permet pas d'affiner l'analyse optique en fonction de la nature de l'échantillon.

Le dispositif du US 2008/252881 est conçu, quant à lui, pour des analyses en mode dynamique et continu d'un flux de liquide. Or cette méthode n'est pas adaptée à des mesures poussées d'échantillons de pétrole brut de grande viscosité.

Ainsi, même si les bases théoriques de ce mode de détection sont à ce jour bien établies, il n'existe aucun matériel pour la mise en oeuvre de cette méthode qui soit capable de déterminer avec précision l'apparition et/ou les taux de matières solides dans des fluides pétroliers de densités extrêmes (huiles lourdes).

En effet, pour ces fluides spécifiques, la densité est telle que, même avec de faibles volumes d'échantillons et de fortes puissances lumineuses, la précision des mesures se révèle largement insuffisante.

La présente invention a pour but de résoudre ces problèmes techniques de manière satisfaisante en proposant une solution permettant d'offrir une fiabilité et une précision des mesures de la quantité de matières solides, quels que soient les volumes et les densités ou viscosités des échantillons.

Ce but est atteint, selon l'invention, au moyen d'une cellule **caractérisée en ce que** les parois de ladite chambre sont pourvues d'au moins un alésage destiné à recevoir, de façon étanche, un élément de mesure optique déplaçable à l'intérieur de ladite chambre entre une position d'escamotage en retrait dans l'alésage pour permettre le passage du piston et une position de mesure dans laquelle il est en saillie dans ladite chambre.

Selon une caractéristique avantageuse, ledit élément de mesure comprend un tronçon de fibre optique raccordé, en amont, à une source laser et coopérant, en aval, avec un détecteur.

Selon une autre caractéristique avantageuse, l'extrémité interne de l'élément de mesure est raccordée à un collimateur.

De préférence, ledit élément de mesure est logé dans une gaine munie, d'une part, d'un organe d'étanchéité périphérique et d'un organe d'étanchéité interne et emprisonnée, d'autre part, dans un mandrin externe de support.

Selon une première variante de l'invention, ledit organe d'étanchéité périphérique est constitué d'une série de bagues déformables.

Selon une autre variante, ledit organe d'étanchéité interne est constitué d'un brasage du rebord de la gaine.

Selon une caractéristique spécifique, au moins l'un desdits mandrins est mobile en rotation dans un filetage du cylindre de façon à assurer le déplacement en translation de l'élément de couplage associé.

De préférence, ledit mandrin est solidaire d'une molette de réglage manuel.

Selon une variante spécifique, ledit alésage est orienté selon un axe diamétral du cylindre.

Selon une autre variante, la cellule comprend deux alésages en regard destinés chacun à recevoir un élément de mesure optique, le premier élément formant émetteur tandis que le second élément forme un récepteur.

Dans ce cas, au moins l'un des deux éléments de mesure est déplaçable en translation en regard de l'autre élément.

Selon encore une autre variante, la paroi d'extrémité longitudinale de la chambre est constituée d'un hublot de visualisation.

Un autre objet de l'invention est un procédé de détection de la présence et/ou de mesure de la quantité de particules solides ou de mesure de la quantité de matières solides dans un échantillon hydrocarbure en utilisant une cellule d'analyse du type défini ci-dessus.

Cette cellule permet aussi de mettre en oeuvre simultanément une étape du procédé consistant à déterminer les conditions de saturation de l'échantillon fluide, appelées plus communément « point de bulle ».

La cellule de l'invention offre des moyens fiables et efficaces de mesure pour des échantillons d'hydrocarbures très variés et dans différentes contenances (de 10 à 2000 cm³).

Bien que la pression régnant dans la chambre de compression soit très élevée (environ 150 MPa) et malgré la présence dans sa paroi d'au moins un alésage, l'étanchéité n'est pas dégradée et est, au contraire, maintenue à son plus haut niveau grâce aux deux organes d'étanchéité respectivement périphérique et interne.

La présence de ces alésages dans la paroi latérale du cylindre permet de conserver le profil cylindrique de la chambre de compression. Cette disposition laisse toute liberté au piston dans sa course et facilite l'agitation de l'échantillon fluide dans la chambre.

Il est ainsi possible d'atteindre les niveaux de pression très élevés dans la chambre qui sont nécessaires pour les mesures dites « PVT » au travers du hublot de visualisation et sans créer de volume mort.

La présence d'un hublot, permet, en outre, de procéder simultanément aux mesures « PVT » et aux mesures optiques sans générer une quelconque interaction.

Enfin, la possibilité de déplacer les éléments de mesure pour les placer au coeur de l'échantillon ou pour les rapprocher mutuellement, permet d'analyser avec une grande précision les fluides pétroliers très denses dont l'opacité était susceptible de constituer un facteur d'erreur dans les mesures.

L'invention sera mieux comprise à la lecture de la description qui va suivre, accompagnée des dessins sur lesquels ;
La figure 1 représente une vue schématique partielle en coupe transversale d'un mode de réalisation de la cellule de l'invention.
Les figures 2A et 2B représentent des vues en coupe longitudinale partielle du mode de réalisation de la figure 1 avec les éléments de détection optique, respectivement, en position d'escamotage et en position de mesure.

La cellule d'analyse représentée sur les figures est destinée à la détermination des propriétés thermodynamiques des hydrocarbures et, plus particulièrement, à l'étude de leur comportement sous des conditions de pression, température et volume (PVT) extrêmes ainsi que la détection et/ou la mesure de la quantité de particules solides présente dans ces hydrocarbures sous ces mêmes conditions. Elle comprend un corps cylindrique 1 dans lequel est logé un piston (non représenté) mobile en translation axiale qui délimite, avec les parois d'extrémité du cylindre, une chambre de compression 10 destinée à recevoir, de façon confinée, un échantillon de l'hydrocarbure à étudier.

La chambre 10 est conçue de façon à pouvoir supporter des pressions de l'ordre de 150 MPa et des températures de l'ordre de 250 °C. La structure du cylindre 1 et notamment l'épaisseur de ses parois 12 est dimensionnée en conséquence.

La cellule est équipée, de façon traditionnelle, de tous les moyens (moteurs, vérins, pompes d'injection et d'extraction, capteurs, calculateurs,...) permettant, notamment, le déplacement du piston, l'introduction de l'échantillon dans la cellule ainsi que le réglage et le contrôle des paramètres thermodynamiques.

Ces moyens, bien que nécessaires au fonctionnement de la cellule, sont traditionnels et ne constituent pas l'objet de l'invention.

La paroi latérale 12 du cylindre 1 est pourvue, au niveau de la chambre 10, au-dessus de la position de repos du piston, d'au moins un alésage 11 et, dans la variante représentée, de deux alésages 11a, 11b en regard.

Ces alésages traversent la paroi 12 du cylindre 1 de part en part et sont destinés à recevoir chacun, de façon étanche, un élément de mesure optique 2.

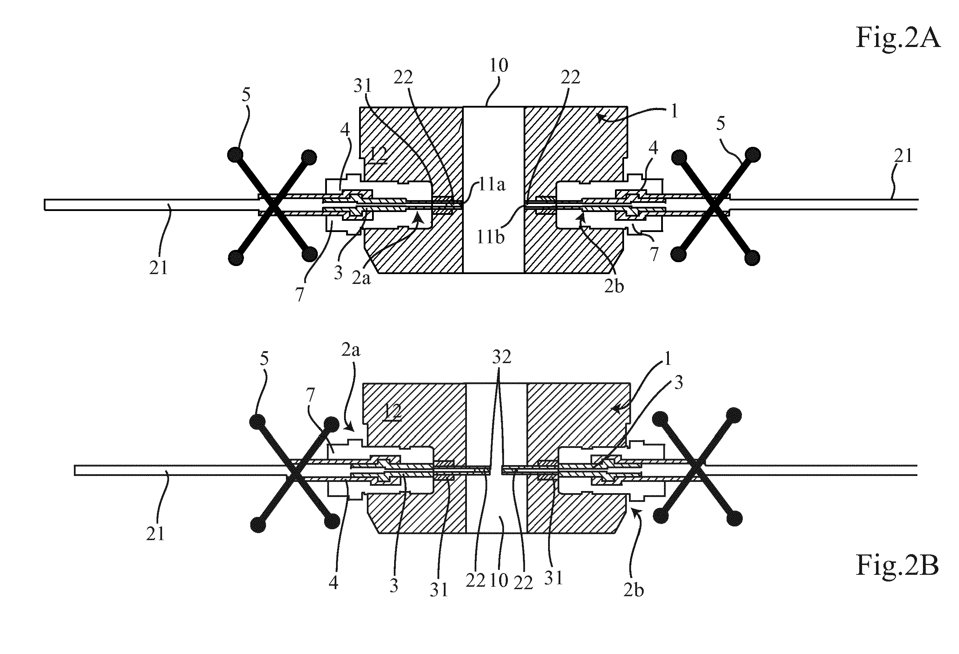

Au moins l'un des éléments de mesure 2 est déplaçable à l'intérieur de la chambre 10 entre une position d'escamotage en retrait dans l'alésage 11 pour permettre le passage du piston (figure 2A) et une position de mesure dans laquelle il vient en saillie dans ladite chambre (figure 2B).

Le caractère télescopique de l'élément de mesure permet de régler la position de l'extrémité active de ce dernier dans la chambre et de la placer à un emplacement optimal pour les mesures au coeur de l'échantillon.

Ces éléments de détection et/ou de mesure 2 comprennent, de façon traditionnelle et comme représenté sur les figures, un tronçon de fibre optique 21 raccordé, en amont, à une source laser (non représentée) et coopérant, en aval, avec un détecteur (non représenté).

L'extrémité interne des éléments de mesure, et donc ici de la fibre, est raccordée optiquement à un collimateur 22 en saphir.

Un microscope électronique peut avantageusement compléter cet équipement, en particulier, pour des opérations de mesure spécifique de la quantité de matières solides.

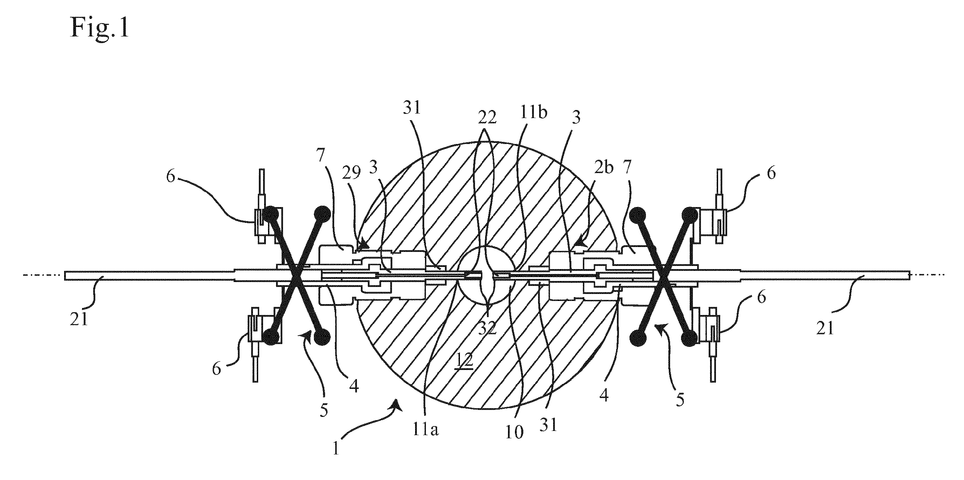

Dans la variante de cellule représentée sur les figures 1, 2A et 2B, il est prévu que les deux éléments de mesure 2a, 2b soient couplés mutuellement. Au moins l'un des deux éléments 2a, 2b et, de préférence les deux éléments, sont déplaçables, en translation sur leur axe commun qui est ici confondu avec le diamètre de la chambre.

Dans cette variante, les collimateurs 22 respectifs de chaque élément 2a, 2b se font donc face.

Le premier élément 2a forme un émetteur tandis que le second élément 2b forme un récepteur. Lors de la phase de détection, le faisceau laser guidé par la fibre 21 passe alors dans le collimateur 22 de l'élément 2a où il est orienté vers la chambre 10 puis au travers de l'échantillon hydrocarbure fluide en éclairant les particules solides en suspension dont la présence et/ou la concentration est déterminée par la mesure des phénomènes de diffraction et d'absorption du signal optique à la réception dans l'élément 2b.

Cette disposition présente un intérêt pour l'analyse de fluide pétroliers de fortes densités et donc de grande opacité pour lesquels l'optimisation et la précision des mesures optiques nécessitent le rapprochement de l'émetteur du récepteur.

Dans le cas où la cellule ne comprend qu'un seul élément de mesure 2, il est possible de prévoir que la paroi de la chambre 10 en regard présente une zone de détection ou de réflexion coopérant avec le faisceau transmis.

Dans cette variante, l'unique élément de mesure pourrait alors intégrer un émetteur et un détecteur du signal optique réfléchi logés dans le même alésage.

Chaque élément de mesure 2 est logé dans une gaine 3 munie, d'une part, d'un organe d'étanchéité périphérique 31 et d'un organe d'étanchéité interne 32 et emprisonnée, d'autre part, dans un mandrin externe 4 de support.

Le mandrin 4 est lui-même engagé dans un raccord haute pression 7 qui est fixé dans une cavité de la paroi 12 du corps de la cellule.

L'organe d'étanchéité périphérique 31 est constitué d'une série de bagues déformables jointives.

L'organe d'étanchéité interne 32 est constitué, quant à lui, d'un brasage du rebord de la gaine 3, à la liaison avec la fibre 21 et/ou le collimateur 22 qui se trouve lui aussi emprisonné à l'intérieur de la gaine.

Le mandrin 4 de support est mobile en rotation dans un filetage ménagé dans le raccord 7 de façon à assurer le déplacement en translation de l'élément de mesure 2 correspondant.

Ce mandrin 4 est solidaire d'une molette 5 de réglage manuel associée à un jeu de butées permettant de s'assurer de l'atteinte de la position d'escamotage de l'élément de mesure 2, en fin de course de recul du mandrin.

Le jeu de butée est actionné par une liaison mécanique travaillant par contact avec le mandrin de façon classique ou par liaison électrique/électronique via un détecteur 6 de la position du mandrin 4 (voir figure 1).

Dans une variante non représentée, il est possible de prévoir que le déplacement télescopique de l'élément de mesure soit assuré par glissement dans un conduit.

La paroi d'extrémité longitudinale de la chambre 10 est obturée par un hublot de visualisation (non représenté) en quartz ou saphir, transparent à la lumière visible et raccordé de façon étanche à la paroi latérale du cylindre 1.

Cet hublot permet d'effectuer, en parallèle et simultanément au procédé de l'invention relatif à la détection des matières solides, des mesures des conditions de saturation utilisant en complément, par exemple, un endoscope éventuellement associé à une caméra vidéo.

## Revendications

1. Cellule d'analyse sous très haute pression et température élevée d'échantillons de fluide comprenant un cylindre (1) dans lequel est logé un piston mobile en translation axiale qui délimite, avec les parois d'extrémité du cylindre, une chambre (10) de compression fermée dans laquelle est confiné l'échantillon, **caractérisée en ce que** les parois de ladite chambre (10) sont pourvues d'au moins un alésage (11) destiné à recevoir, de façon étanche, un élément de mesure optique (2) déplaçable à l'intérieur de ladite chambre entre une position d'escamotage en retrait dans l'alésage pour permettre le passage du piston et une position de mesure dans laquelle il est en saillie dans ladite chambre.

2. Cellule d'analyse selon la revendication 1, **caractérisée en ce que** ledit élément de mesure (2) comprend un tronçon de fibre optique (21) raccordé, en amont, à une source laser et coopérant, en aval, avec un détecteur.

3. Cellule d'analyse selon la revendication 1 ou 2, **caractérisée en ce que** l'extrémité interne de l'élément de mesure (2) est raccordée à un collimateur (22).

4. Cellule d'analyse selon l'une des revendications précédentes, **caractérisée en ce que** ledit élément de mesure (2) est logé dans une gaine (3) munie, d'une part, d'un organe d'étanchéité périphérique (31) et d'un organe d'étanchéité interne (32) et emprisonnée, d'autre part, dans un mandrin externe (4) de support.

5. Cellule d'analyse selon la revendication 4, **caractérisée en ce que** ledit organe d'étanchéité périphérique (31) est constitué d'une série de bagues déformables.

6. Cellule d'analyse selon la revendication 4 ou 5, **caractérisée en ce que** ledit organe d'étanchéité interne (32) est constitué d'un brasage du rebord de la gaine (3).

7. Cellule d'analyse selon l'une des revendications 4 à 6, **caractérisée en ce que** ledit mandrin (3) est mobile en rotation dans un filetage ménagé dans la paroi du cylindre (1) de façon à assurer le déplacement en translation de l'élément de mesure (2).

8. Cellule d'analyse selon la revendication 7, **caractérisée en ce que** ledit mandrin (4) est solidaire d'une molette (5) de réglage manuel.

9. Cellule d'analyse selon l'une des revendications précédentes, **caractérisée en ce que** ledit alésage est orienté selon un axe diamétral du cylindre (1).

10. Cellule d'analyse selon l'une des revendications précédentes, **caractérisée en ce qu'**elle comprend deux alésages (11a, 11b) en regard destinés chacun à recevoir un élément de mesure optique (2a, 2b), le premier élément (2a) formant émetteur tandis que le second élément (2b) forme un récepteur.

11. Cellule d'analyse selon la revendication 10, **caractérisée en ce qu'**au moins l'un des deux éléments de mesure (2a, 2b) est déplaçable en translation en regard de l'autre élément.

12. Cellule d'analyse selon l'une des revendications précédentes, **caractérisée en ce que** la paroi d'extrémité longitudinale de la chambre (10) est constituée d'un hublot de visualisation (12).

13. Procédé de détection et/ou de mesure de la quantité de matières solides présente dans un échantillon hydrocarbure introduit dans une cellule d'analyse selon l'une des revendications précédentes, **caractérisé en ce qu'**on déplace l'élément de mesure (2) à l'intérieur de la chambre (10) depuis une position d'escamotage en retrait dans l'alésage (11) pour permettre le passage du piston vers une position de mesure dans laquelle ledit élément (2) vient en saillie dans ladite chambre puis on règle la position de l'extrémité active de l'élément (2) pour la placer au coeur de l'échantillon.

14. Procédé selon la revendication 13 **caractérisé en ce qu'**on détermine simultanément les conditions de saturation et le point de bulle de l'échantillon.

## Patentansprüche

1. Analysezelle für Fluidproben unter sehr hohem Druck und hoher Temperatur, die einen Zylinder (1) umfasst, in dem ein in axialer Verschiebung bewegbarer Kolben untergebracht ist, der mit den Endwänden des Zylinders eine geschlossene Kompressionskammer (10) begrenzt, in der die Probe eingeschlossen ist, **dadurch gekennzeichnet, dass** die Wände der Kammer (10) mit mindestens einer Bohrung (11) ausgestattet sind, die dazu bestimmt ist, ein optisches Messelement (2) dicht aufzunehmen, das im Innern der Kammer zwischen einer eingezogenen Position, zurückgezogen in der Bohrung um den Durchgang des Kolbens zu erlauben, und einer Messposition, in der es in die Kammer hineinragt, verlagerbar ist.

2. Analysezelle nach Anspruch 1, **dadurch gekennzeichnet, dass** das Messelement (2) einen Abschnitt einer optischen Faser (21) umfasst, der stromaufwärts an eine Laserquelle angeschlossen ist und stromabwärts mit einem Detektor zusammenarbeitet.

3. Analysezelle nach Anspruch 1 oder 2, **dadurch gekennzeichnet, dass** das innere Ende des Messelements (2) an einen Kollimator (22) angeschlossen ist.

4. Analysezelle nach einem der vorangehenden Ansprüche, **dadurch gekennzeichnet, dass** das Messelement (2) in einer Hülle (3) untergebracht ist, die einerseits mit einem peripheren Dichtigkeitsorgan (31) und einem internen Dichtigkeitsorgan (32) ausgestattet und andererseits von einem externen Stützfutter (4) umfasst ist.

5. Analysezelle nach Anspruch 4, **dadurch gekennzeichnet, dass** das periphere Dichtigkeitsorgan (31) von einer Reihe (!) verformbarer Ringe gebildet ist.

6. Analysezelle nach Anspruch 4 oder 5, **dadurch gekennzeichnet, dass** das interne Dichtigkeitsorgan (32) von einer Lötung der Kante der Hülle (3) gebildet ist.

7. Analysezelle nach einem der Ansprüche 4 bis 6, **dadurch gekennzeichnet, dass** das Futter (3) in einem in die Wand des Zylinders (1) eingearbeiteten Gewinde rotierend bewegbar ist, um die verschiebende Verlagerung des Messelements (2) zu sichern.

8. Analysezelle nach Anspruch 7, **dadurch gekennzeichnet, dass** das Futter (4) mit einem manuellen Einstellrädchen (5) verbunden ist.

9. Analysezelle nach einem der vorangehenden Ansprüche, **dadurch gekennzeichnet, dass** die Bohrung gemäß einer diametralen Achse des Zylinders (1) ausgerichtet ist.

10. Analysezelle nach einem der vorangehenden Ansprüche, **dadurch gekennzeichnet, dass** sie zwei gegenüberliegende Bohrungen (11a, 11b) umfasst, die jeweils zur Aufnahme eines optischen Messelements (2a, 2b) bestimmt sind, wobei das erste Element (2a) einen Sender bildet, wogegen das zweite Element (2b) einen Empfänger bildet.

11. Analysezelle nach Anspruch 10, **dadurch gekennzeichnet, dass** mindestens eines der zwei Messelemente (2a, 2b) im Verhältnis zum anderen Element verschiebend verlagerbar ist.

12. Analysezelle nach einem der vorangehenden Ansprüche, **dadurch gekennzeichnet, dass** die Längsendwand der Kammer (10) von einem Sichtfenster (12) gebildet ist.

13. Detektions- und/oder Messverfahren der Menge festen Materials in einer Kohlenwasserstoffprobe in einer Analysezelle nach einem der vorangehenden Ansprüche, **dadurch gekennzeichnet, dass** das Messelement (2) in der Kammer (10) von einer eingezogenen Position, zurückgezogen in der Bohrung (11), um den Durchgang des Kolbens zu erlauben, in eine Messposition, in der das Element (2) in die Kammer hineinragt, verlagert wird und danach die Position des aktiven Endes des Elements (2) eingestellt wird, um es im Zentrum der Probe zu platzieren.

14. Verfahren nach Anspruch 13, **dadurch gekennzeichnet, dass** die Sättigungsbedingungen und der Blasenpunkt der Probe gemeinsam bestimmt werden.

## Claims

1. An analysis cell for the analysis, at very high pressure and high temperature, of fluid samples comprising a cylinder (1) in which an axially translatable piston is housed that defines, with the end walls of the cylinder, a closed compression chamber (10) in which the sample is confined, **characterized in that** the walls of said chamber (10) are provided with at least one bore (11) designed for sealably receiving an optical measuring element (2) movable inside said chamber between a retracted position retracted in the bore to allow the piston to pass, and a measuring position in which it protrudes in said chamber.

2. The analysis cell according to claim 1, **characterized in that** said measuring element (2) comprises an optical fiber segment (21) connected upstream to a laser source and cooperating downstream with the detector.

3. The analysis cell according to claim 1 or 2, **characterized in that** the inner end of the measuring element (2) is connected to a collimator (22).

4. The analysis cell according to one of the preceding claims, **characterized in that** said measuring element (2) is housed in a sheath (3) provided on the one hand with a peripheral sealing member (31) and an inner sealing member (32) and, on the other hand, captured in an outer support mandrel (4).

5. The analysis cell according to claim 4, **characterized in that** said peripheral sealing member (31) is formed by a series of deformable rings.

6. The analysis cell according to claim 4 or 5, **characterized in that** said inner sealing member (32) is formed by brazing the rim of the sheath (3).

7. The analysis cell according to one of claims 4 to 6, **characterized in that** said mandrel (3) is rotatable in a threading formed in the wall of the cylinder (1) so as to ensure the translation of the associated measuring element (2).

8. The analysis cell according to claim 7, **characterized in that** said mandrel (4) is secured to a manual adjustment wheel (5).

9. The analysis cell according to one of the preceding claims, **characterized in that** said bore is oriented in a diametrical axis of the cylinder (1).

10. The analysis cell according to one of the preceding claims, **characterized in that** it comprises two opposite bores (11a, 11b) each designed to receive an optical measuring element (2a, 2b), the first element (2a) forming a transmitter while the second element (2b) forms a receiver.

11. The analysis cell according to claim 10, **characterized in that** at least one of the two measuring elements (2a, 2b) is translatable across from the other element.

12. The analysis cell according to one of the preceding claims, **characterized in that** the longitudinal end wall of the chamber (10) is made up of a viewing porthole (12).

13. A method for detecting the presence and/or measuring the quantity of solid particles or measuring the quantity of solid matter in a hydrocarbon sample introduced in an analysis cell according to any one of the preceding claims, **characterized in that** the optical measuring element (2) is first moved inside the chamber (10) between a retracted position retracted in the bore (11) to a measuring position in which said element (2) protudes in said chamber and then the position of the active end of said element (2) is adjusted to place it at the heart of the sample.

14. The method according to claim 13, **characterized in that** the saturation conditions and the bubble point of the sample are determined simultaneously.
